# EUROPEAN PATENT APPLICATION

(11) **EP 4 614 405 A2**
(43) Date of publication of application: **10.09.2025**
(21) Application number: 25169880.9
(22) Date of filing: 28.04.2017
(51) Int. Cl.: G06N 20/10

(54) **MACHINE LEARNING ALGORITHM**

(30) Priority: 29.04.2016 GB 201607521
(62) Divisional of application: 17726197.1
(71) Applicant: NEC OncoImmunity AS, 0379 Oslo (NO)
(72) Inventor: STRATFORD, Richard, 0379 Oslo (NO); CLANCY, Trevor, 0379 Oslo (NO)
(74) Representative: Gill Jennings & Every LLP

(57) **Abstract**

The present invention provides a method for identifying peptides that contain features positively associated with natural endogenous or exogenous cellular processing, transportation and major histocompatibility complex (MHC) presentation. In particular, the invention/method controls for the influence of protein abundance, stability and HLA/MHC binding on processing and presentation, enabling a machine-learning algorithm or statistical inference model trained using the method to be applied to any test peptide regardless of its HLA/MHC restriction i.e. the algorithm operates in a HLA/MHC-agnostic manner. This is attained through the building of positive and negative data sets of peptide sequences (peptides identified or inferred from surface bound or secreted MHC/peptide complexes in the literature, and those which are not). Specifically, the positive and negative data sets comprise a multiplicity of pairings between individual entries, in which both sequences of a pair are of equal or similar length, and are derived from the same source protein, and/or have similar binding affinities, with respect to the HLA/MHC molecule from which the peptide of the positive peptide is restricted.

## Description

### Field of the Invention

The present invention relates to methods of identifying peptides that contain features associated with successful cellular processing, transportation and major histocompatibility complex presentation, through the use of a machine learning algorithm or statistical inference model.

### Background to the Invention

The identification of immunogenic antigens from pathogens and tumours has played a central role in vaccine development for decades. Over the last 15-20 years this process has been simplified and enhanced through the adoption of computational approaches that reduce the number of antigens that need to be tested. While the key features that determine immunogenicity are not fully understood, it is known that most immunogenic class I peptides (antigens) are generated in the classical pathway through proteasomal cleavage of their parental polypeptide/protein in the cytosol, are subsequently transported into the endoplasmic reticulum by the TAP transporters, before being packaged into empty HLA/MHC molecules and transported to the surface and presented to circulatory CD8+ T-cells.

The ability of a peptide to bind HLA/MHC represents the most important step in determining immunogenicity, as only HLA/MHC-bound peptide can bind and activate circulating T-cells and this area of research has been very active. There are now well-populated publically available databases that list numerous validated HLA/MHC-ligands for the most common HLA/MHC alleles such as the IEDB (http://www.iedb.org/; as accessed in April 2016). These databases have been used to train different types of prediction algorithms which are able to reliably predict whether *de novo* untested peptides can bind to a given allele and attempt to predict the binding affinity with varying degrees of success. However, a significant proportion of the HLA/MHC binding data cited in these databases are from *in vitro* binding studies and thus contain many examples of peptides that are not naturally processed *in vivo.*

Interestingly, recent studies have shown that less than 15% of validated MHC binders are naturally processed and are thus actually observed at the surface of the cell (Giguere et al. 2013). Furthermore, less than 5% of predicted MHC binders are immunogenic i.e. bind and activate a circulating T-cell (Paul F Robbins et al. 2013), demonstrating the important role processing and presentation play in determining immunogenicity. Thus there is a clear need to supplement HLA/MHC prediction algorithms with additional algorithms that have been trained to recognize the key features of a peptide that are synonymous with efficient processing and presentation.

The earliest attempts at developing computational methods for predicting processing & presentation focused on predicting specific steps within the classical pathway such as proteasomal cleavage in the cytosol. For example, FragPredict, ProteaSMM, PAProC & PepCleave have been trained on the *in vitro* proteasome digestion data from β-casein and enolase (Holzhutter and Kloetzel 2000; Tenzer et al. 2005; Nussbaum et al. 2001; Ginodi et al. 2008; Emmerich et al. 2000; & Toes et al. 2001). While NetChop and an updated version of ProteaSMM are trained on the *in vitro* proteasome digestion data from β-casein, enolase, and the prion-protein (Kesmir et al. 2002; Nielsen et al. 2005; Emmerich et al. 2000; Toes et al. 2001; Tenzer et al. 2004). However, while these methods have proven to be reasonably accurate at predicting the cleavage patterns observed in novel *in vitro* proteasome digestion experiments, they are not very good at predicting MHC-I ligands identified from peptide elution studies. This poor performance probably reflects the fact that the proteolytic activity of proteasomes *in vitro* may not reflect their *in vivo* activity, and that proteasome digestion represents only one step in the complex processing and presentation pathway.

An alternative and potentially more holistic approach which captures the activity of other proteases that contribute to *in vivo* proteolysis (in addition to the proteasome) was described by Kesmir et al, 2002, and infers *in vivo* cleavage sites from non-redundant MHC I ligands. The authors of the method assigned the C-terminus of positive peptides (MHC I ligands) as cleavage sites, and assigned the remaining positions within the same ligand as negative sites (as they must have survived the proteolytic activity in the cytosol & endoplasmic reticulum), and used the data to train a neural-network based machine-learning algorithm called NetChop-Cterm. While NetChop-Cterm performs relatively well with cleavage/non-cleavage data-sets generated using the same principles, it has not been particularly successful at identifying immunogenic epitopes. For example, studies combining an earlier version of NetChop (NetChop-2) and HLA/MHC-binding predictions did not significantly improve epitope prediction compared to the use of HLA/MHC-binding predictions in isolation (Nielsen et al, 2005). One possible explanation for this lack of synergy with HLA/MHC-binding predictors is the fact that the approach of selecting negative cleavage sites by default creates a large binding affinity differential between the positive and negative data sets. This imbalance in the training set is likely to generate algorithmic performance that has learned features of both protease cleavage and HLA/MHC binding, rather than processing features *per se.* Thus the two predictors are by and large performing overlapping tasks and thus not synergistic.

More recently, a number of more holistic computational approaches for predicting processing & presentation have been developed such as MHC-NP & NIEluter that are not focused on an individual step, but instead try to learn all the features that are relevant to the endogenous processing and presentation pathway (Sebastien Giguere et al. 2013 & Qiang Tang et al. 2014). Both these approaches used training and testing data sets for six human HLA/MHC alleles (HLA-A*02:01, HLA-B*07:02, HLA-B*35:01, HLA-B*44:03, HLA-B*53:01 and HLA-B*57:01) that were provided as part of the 2012 second machine learning completion in immunology hosted by the Brusic team at Dana-Farber Cancer Institute. The aim of the competition was to distinguish naturally processed peptides from peptides that are not naturally processed. Both MHC-NP & NIEluter use support vector machine based classifiers trained on bone-fide HLA/MHC eluted peptides identified in peptide elution assays (positive data set), and either validated HLA/MHC binding peptides (a minority of which will be naturally processed) and/or peptides that have been shown not to bind the HLA/MHC molecule in *in vitro* binding studies.

Whilst both MHC-NP & NIEluter report good performances when tested against the test sets provided, scrutinizing both the training and test sets identifies a significant binding affinity differential between the positive and negative datasets. This binding differential is likely to generate algorithms that have learnt features of both processing and HLA/MHC binding, rather than processing features *per se,* and in addition the HLA/MHC-restricted nature of these tools limits their utility in antigen discovery.

There therefore exists a need in the art for an approach which exclusively identifies the key features determining processing and presentation. Moreover, it is highly desirable to be able to offer accurate predictions for any peptide regardless of its MHC restriction.

### Summary of the Invention

The present invention provides a method for identifying peptides which contain features that are positively associated with successful navigation of the cell's natural endogenous and/or exogenous processing, transportation and presentation pathway. Thus these peptides if they are capable of binding a specific MHC molecule, are likely to be detectable on the surface of the cell in a MHC-peptide (MHC-p) complex.

This is achieved by applying a machine learning algorithm or statistical inference model on a training data set comprising a positive and a negative data set, built in the manner defined herein. The positive data set comprises entries of peptide sequences identified or inferred from surface bound or secreted MHC-p complexes; notably via peptide elution assays reported in the literature. The negative data set comprises entries of sequences for which said identification or inference has not been reported.

The training data further comprise a multiplicity of pairings between entries of the positive and negative data sets. Both sequences in each pair are of equal or similar length, and are either derived from the same source protein (or fragment thereof) and/or have comparable estimated binding affinities with respect to the HLA/MHC molecule which the positive member of the pair is reportedly restricted (forms a complex with).

Through the use of sequences as training data which are preferably identified or inferred from surface bound or secreted HLA/MHC molecules encoded by a plurality of HLA/MHC alleles, and the creation of negative pairs with comparable HLA/MHC binding affinities to their positive counterparts, and/or the removal of amino acids at key HLA/MHC-binding anchor positions, the method controls for the influence of HLA/MHC-binding on the efficiency of the processing and presentation pathway, and ensures that the algorithm learns features associated with efficient processing and presentation rather than HLA/MHC binding. Therefore, for the example of processing and presentation by human leukocyte antigen (HLA) molecules, the invention is considered "HLA-agnostic". Thus, an algorithm trained with the method may be used to make accurate predictions for any known or predicted HLA - p complex, and is not limited to those encoded by a specific HLA allele or a specific HLA gene loci, although the method can be applied to train a machine learning algorithm or statistical inference model on training data identified or inferred from a HLA molecule encoded by a single allele. Such a trained machine learning algorithm or statistical inference model can therefore be used to make HLA/MHC allele-specific predictions. Furthermore by selecting the negative sequence of the pair from the same source protein as the positive counterpart, the method controls for differences in parental protein expression and stability and reduces the risk of introducing false negatives i.e. peptides that contain excellent processing features but are not observed at the surface of the cell complexed with HLA/MHC as the parental protein exhibits sub-optimal expression and/or stability characteristics required for MHC/HLA presentation. This leads to improved training data and more accurate predictions

Accordingly, in a first aspect, the invention provides a method for training a machine learning algorithm or statistical inference model to identify peptides that contain features positively associated with natural endogenous or exogenous cellular processing, transportation and HLA/MHC presentation; that negates the influence of HLA/MHC-binding and can be applied to any peptide regardless of its HLA/MHC restriction, comprising:
(a) building one or more training data sets comprising a positive and a negative data set;
   wherein the positive data set comprises entries of peptide sequences identified or inferred from surface bound or secreted HLA/MHC-p complexes encoded by one or a plurality of different HLA/MHC alleles, and wherein the negative data set comprises entries of peptide sequences which are not identified or inferred from surface bound or secreted HLA/MHC-p complexes;
   wherein the training data further comprises a multiplicity of pairings between entries of the positive and negative data sets; and wherein each pair of said multiplicity of pairings comprises peptide sequences which:
      (i) are of equal or similar length,
         and
      (ii) are derived from the same source protein (or fragment thereof), and/or
      (iii) have similar binding affinities, with respect to the HLA/MHC molecule which the peptide of the positive data set is restricted..
and (b) applying a machine learning algorithm or statistical inference model on said training data.

According to a second aspect, the invention provides a computer readable medium having computer executable instructions stored thereon for implementing the method of the first aspect.

According to a third aspect, the invention provides an apparatus comprising:
one or more processors; and
memory comprising instructions which when executed by one or more of the processors cause the apparatus to perform the method of the first aspect.

Further aspects are defined in the Detailed Description of the Invention.

### Brief Description of the Figures

Figure 1 demonstrates that selecting the negative peptide from the same protein as the positive peptide versus a random protein when building the training data improves the predictive performance of the algorithm.
Figure 2 demonstrates how changes in the binding differential between the positive and negative matched pairs used to construct the training data influences the performance of the algorithm.
Figure 3 demonstrates the optimal criteria for selecting the negative peptides for both strong (IC50 =< 500) and weak (IC50 < 500) binders.
Figure 4 demonstrates the HLA/MHC-agnostic nature of algorithms trained using the method described herein i.e. the algorithm can correctly classify novel peptides isolated from HLA/MHC alleles that were not represented in the original training data.
Figure 5 demonstrates the superior performance of a SVM algorithm trained using the method described herein versus the best performing HLA/MHC-agnostic classifier published in the literature called NetChop-Cterm-3.0.
Figure 6 demonstrates the superior performance of a SVM algorithm trained using the method described herein versus one of the best performing allele-specific-trained SVM-based classifiers "MHC-NP" which was trained on data sets provided by the Brusic team at Dana-Farber Cancer Institute as part of the 2012 second machine learning completion in immunology.

### Detailed Description of the Invention

All terminology used herein has the standard definition used in the art, unless otherwise indicated.

According to a first aspect, the invention provides a method for training a machine learning algorithm or statistical inference model to identify peptides that contain features positively associated with natural endogenous or exogenous cellular processing, transportation and HLA/MHC presentation; that negates the influence of HLA/MHC-binding and can be applied to any peptide regardless of its HLA/MHC restriction, comprising:
(a) building one or more training data sets comprising a positive and a negative data set;
   wherein the positive data set comprises entries of peptide sequences identified or inferred from surface bound or secreted HLA/MHC-p complexes encoded by one or a plurality of different HLA/MHC alleles, and wherein the negative data set comprises entries of peptide sequences which are not identified or inferred from surface bound or secreted HLA/MHC-p complexes;
   wherein the training data further comprises a multiplicity of pairings between entries of the positive and negative data sets; and wherein each pair of said multiplicity of pairings comprises peptide sequences which:
      (i) are of equal or similar length,
         and
      (ii) are derived from the same source protein (or fragment thereof), and/or
      (iii) have similar binding affinities, with respect to the HLA/MHC molecule which the peptide of the positive data set is restricted
and (b) applying a machine learning algorithm or statistical inference model on said training data.

In fields where the exact mechanisms of a process have not been fully developed, machine learning systems are particularly beneficial, as they can perform pattern recognition and learning techniques on existing data sets to build predictive models. Where it is known that certain inputs result in desired outcomes, and other inputs result in undesirable outcomes, machine learning systems can identify what parameters of those inputs may be indicative of desirable and undesirable outcomes, thereby providing a predictive model without any fundamental understanding of the mechanisms involved.

Machine learning systems need to be trained on existing data, known as training data, in order to build the machine learning model. The choice of training data can have a significant impact on the effectiveness of a trained machine learning algorithm, and the claimed solution provides a particularly effective teaching of what training data should be used for developing an improved machine learning model.

In accordance with an example embodiment of the proposed solution, matched pairs may be provided as training data to the machine learning system. Each pairing may be a peptide sequence with the desired outcome (positive data) and a peptide sequence with the undesired outcome (negative data). Each of the positive and negative data may include one or more parameters defining characteristics of the peptide sequences, and the machine learning algorithm can be trained to determine what combinations of parameters can result in desired outcomes under different conditions.

Each peptide sequence, for example, may be represented as a feature vector, which is an n-dimensional vector of numerical parameters that represent that peptide sequence. The feature vectors of positive data may be stored in one data structure, and the feature vectors of negative data may be stored in another data structure, and a separate data structure may provide linkages between matching pairs of the feature vectors of the positive and negative data. Alternatively, the matched pairs of positive and negative data may be stored in a single data structure, such as a set of two-tuples wherein the first element of the two-tuple is an n-dimensional feature vector of a positive peptide sequence, and the second element of the two-tuple is an n-dimensional feature vector of a negative peptide sequence. In some embodiments, the peptide sequences are represented as concatenated vectors, wherein each amino acid is encoded as a binary vector with one element for each possible amino acid, and wherein the presence of each amino acid is denoted with a 1 and the absence of each amino acid is denoted with a 0. As defined herein, "binary vector" or "bit array" refers to a data structure that compactly stores bits or binary values, where each element, or bit, of the vector can be represented by only a binary value, for example, 0 or 1.

There are several different implementations of machine learning available, and the skilled person would be able to adapt the implementation used depending on features such as the data sets available, the processing power available, and the accuracy desired. The skilled person may choose to include as many parameters in each feature vector as possible, to improve the accuracy of the data model. Alternatively, the skilled person may choose fewer parameters to reduce the computational complexity of the task.

The machine learning system is preferably distributed over several logically connected computer systems to satisfy the large computational requirements for performing machine learning on large data sets, but the machine learning system may be implemented on a single computer system.

In accordance with the first aspect, it is necessary to construct the positive data set using entries of peptide sequences identified or inferred from surface bound or secreted HLA/MHC-peptide complexes. Typically, combined sets of positive peptides may be used which have been identified experimentally in the literature, for example HLA/MHC "peptidomes" reported for a specific cell type (as taught in, for example, Espinosa et al. (2013) and Jarmalavicius et al. (2012) - see present Example). The positive dataset may be constructed using entries of peptide sequences identified or inferred to be surface bound or secreted with a HLA/MHC molecule encoded by a single allele. Preferably, the positive data set (and/or the complementary negative dataset) comprises peptide sequences identified from multiple different cell lines or primary cells which express various different HLA/MHC alleles. In this embodiment, said positive and/or negative data sets comprise peptide sequences identified or inferred from surface bound or secreted MHC/HLA-p complexes encoded by a "plurality" of different HLA/MHC alleles, where "plurality" refers to two or more HLA/MHC alleles. Each "peptidome" (or set of positive peptides) will likely have been identified using standard protocols available in the art. These typically comprise cell lysis, purification by affinity chromatography (using antibodies that are either specific for a particular allelic variant of HLA/MHC, or recognise determinants that are common across multiple allelic variants, or an entire class of HLA/MHC) and ultrafiltration, optionally HPLC separation, and subsequently peptide identification by mass spectrometry (for example, matrix-assisted laser desorption ionisation time-of-flight mass spectrometry (MALDI-TOF MS)). For exemplary protocols, see Espinosa et al. (2013), page 25 "2. Materials and methods", or Jarmalavicius et al. (2012), page 33402 "Experimental Procedures".

In accordance with the first aspect, features (i), (ii) and (iii) are to be construed as requiring feature (i), in addition to either one or both of features (ii) and (iii). Preferably, each pair of said multiplicity of pairings consists of two sequences having said features (as construed above). More preferably, each pair of said multiplicity of pairings comprises, more preferably consists of, two sequences having all of features (i), (ii) and (iii).

Concerning feature (i), the sequences are preferably 8, 9, 10, 11 or greater than 11 amino acids in length. Preferably, class I peptides are between 8 and 14 amino acids in length and class II peptide are between 9 and 32 amino acids in length. In this context, "similar" length is within these limits, i.e. for class I peptides, similar length is from 8 to 14 amino acids (up to six amino acids in difference), and for class II peptides similar length is from 9 to 32 amino acids (up to 23 amino acids difference). It is furthermore preferred that each peptide sequence of both the positive and negative data sets is of equal length (i.e. equal lengths are not only present between paired positive and negative entries, but all entries in both data sets).

Concerning feature (ii), this may be determined by the skilled person using databases and search functions available in the art. By way of example, pairs may be constructed by reference to entries of the Uniprot database (The UniProt Consortium; 2014. http://www.uniprot.org/; as accessed in April 2016).

Concerning feature (iii), this is preferably determined *in silico* using known HLA/MHC binding prediction algorithms available in the art. *In vitro* HLA/MHC binding competition assays may be used (possibly in combination with *in silico* methods). Binding affinity is often expressed as an IC₅₀ value measured in nM, which is the concentration of the query peptide predicted to cause 50% inhibition of binding of a standard peptide which is known to bind to a specific HLA/MHC variant with high affinity. However, alternative measurements or comparisons of binding affinity can also be utilised for selecting the matching negative peptide such as the binding percentile *etc.*

For the avoidance of doubt, the binding prediction is performed with respect to the same HLA/MHC molecule from which the positive member of the matching pair was identified or inferred as forming a complex with (otherwise known as "restricted"). If the IC₅₀ metric is used to select the negative member of a matching pair, the IC₅₀ value of the negative peptide should differ by no more than (in increasing preference) 500%, 200%, and 100%, compared to the binding affinity of its positive counterpart.

Further according to said first aspect, it is preferable to the HLA/MHC-agnostic nature of the invention (see Example 4) that the positive data set comprises peptide sequences identified or inferred from a plurality of different HLA/MHC alleles. As detailed above, it is preferred that said sequences are identified or inferred from multiple different tissue samples, cell lines or primary cells, which express different HLA/MHC alleles. Therefore, it is typically necessary to construct a positive data set comprising peptide sequences identified or inferred from multiple different human (or animal) subjects expressing a variety of different HLA/MHC alleles.

It is furthermore preferred that said peptide sequences (of the positive data set) are identified or inferred from surface bound or secreted HLA/MHC molecules encoded by (a) HLA/MHC Class I alleles of either the HLA -A, -B or -C gene loci (or equivalent loci thereof in a non-human species), or any combination thereof; or (b) HLA/MHC class II alleles of either the HLA -DQ, -DP or DR gene loci (or equivalent loci thereof in a non-human species), or any combination thereof; wherein the positive data set is derived from the same species. In some embodiments, said positive data set comprises peptide sequences identified or inferred from all of said gene loci according to (a), or all of said gene loci according to (b). In some embodiments, the non-human species is an animal.

Further according to said first aspect, key HLA/MHC-binding anchor positions within the peptide sequences of the positive and negative data sets can be excluded as features for the machine learning algorithm or statistical inference model. Preferably, said key HLA/MHC-binding anchor positions are positions 2 and 9 of the peptide sequence (for class I HLA/MHC alleles) and anchor positions 1, 4, 6 & 9 (for class II alleles).

Further according to said first aspect, the following are preferably used as features for the machine learning algorithm or statistical inference model:
(1) amino acid identity, size, charge, polarity, hydrophobicity and/or other physicochemical property at any given position in sequences of the positive and negative data sets.
(2) amino acid identity, size, charge, polarity, hydrophobicity and/or other physicochemical property in positions which, in the source protein, are within 10, preferably 5, more preferably 3 positions of the termini of the sequences of the positive and negative data sets (known as peptide flanking regions).
(3) Principle component score vectors of hydrophobic, steric and electronic properties (VHSE) descriptors (Mei et al. 2005) for the amino acids of the sequences of the positive and negative data sets.
(4) Principle component score vectors of topological and structural properties (VTSA) descriptors (by ZhiLiang et al. 2008) for the amino acids of the sequences of the positive and negative data sets.
*(5) k-mer* frequency of an amino acid sequence at any given position in the peptide sequences of the positive and negative data sets; wherein k is equal to 2 or 3.

Any one, combination, or all, of the above may be used as features for the machine learning algorithm or statistical inference model.

Further according to said first aspect, in a further embodiment the method further comprises the interrogation of input data comprising sequences of peptides, whole proteins or fragments thereof. Wherein the input data comprises whole proteins or fragments thereof, such sequences may be broken into peptides of length as defined above, preferably nonameric peptides, prior to testing. The outputs will be classified into one of two categories: processed and presented on the cell surface or not processed or presented on the cell surface, or converted into a probabilistic scale using mathematical techniques such as Platt scaling.

According to a third aspect of the invention, a computer readable medium is provided comprising instructions which when executed by one or more processors of an electronic device, cause the electronic device to operate in accordance with the method as defined in accordance with the method of the first aspect of the invention.

According to a fourth aspect of the present invention, an electronic device is provided comprising: one or more processors; and memory comprising instructions which when executed by one or more of the processors cause the electronic device to operate in accordance with the method of the first aspect of the invention.

According to a fifth aspect of the present invention, there is provided a module for building training data as defined in the method of the first aspect of the invention.

According to a sixth aspect of the present invention, there is provided a module for machine learning in accordance with the method of the first aspect of the invention.

### Materials and Methods - constructing the positive and negative training datasets to remove the influence of protein abundance, stability and HLA/MHC (HLA/MHC) binding.

Naturally processed nonomeric peptides were identified from numerous HLA/MHC/peptide elution studies reported in the scientific literature. These peptides were subsequently filtered according to whether they could be matched to a single source protein by reference to the UniProtKB data base(The UniProt Consortium, 2014).. The single source proteins were then scrutinized using a HLA/MHC binding prediction algorithm to identify other nonomeric peptides with a similar binding affinity (range varied according to the experiment), but which were not observed in any of the peptide elution assays. Thus, matched pairs of positive peptides (identified in an elution assay) and negative peptides (peptides that occurred in the same parental protein as the positive, have a similar predicted binding affinity, but were not observed in any of the elution assays) were developed. The use of matched pairs from the same source protein controls for the fact that differences in protein expression and stability can influence the efficiency of processing and presentation of a peptide in a sequence independent manner i.e. peptides that contain excellent processing features may never be observed at the surface of the cell complexed with HLA/MHC as their parental protein has the wrong expression and stability characteristics. Thus using matched pairs from the same protein ensures that each positive and negative peptide has an equal opportunity to be processed, thus any difference in processing and efficiency should reflect differences in the physiochemical features of each peptide. Secondly, by ensuring both members of a matched pair have equivalent predicted binding affinities, we control for the influence of HLA/MHC-binding on the efficiency of the processing and presentation pathway, and ensure that the algorithm does not erroneously learn the features of the peptide that dictate HLA/MHC binding.

The final training set consisted of 37,648 peptides (18,824 positive peptides & 18,824 negative peptides) isolated from 12 different HLA/MHC-A alleles, 14 different HLA/MHC-B alleles and 5 different HLA/MHC-C alleles.

### Training features

Unless otherwise stated all algorithms were trained using VHSE and frequency vector (dimers) as training features.

### Testing

A number of independent test sets were used to validate the predictive power of the SVM model and compare its performance against other classifiers trained using alternative methods: All of the test sets contain nonomers identified from peptide elution assays with predicted binding affinities of 500nm or less for their respective HLA/MHC allele (except the Sample10 complementary test set - described later). A matching negative test set was then constructed based on the method described above, except the negative peptides were selected on the basis of having a predicted IC₅₀ score within a 10% range of the matched positive peptide (see below). In addition cross validation and conventional validation was performed.

### Independent test sets

### Melanoma test set

Nonomeric class I peptides eluted from four different melanoma cell lines with a predicted IC₅₀ value of 500nm or less (described by Jarmalavicius et al, 2012) were used to generate the positive test set. Matched negatives were then identified from the same parental protein as described above. The final test set contained 206 peptides in total; 103 that were isolated from 5 different class I HLA/MHC alleles and their 103 matched negative partners.

### Thymus test set

Nonomeric class I peptides eluted from human thymic tissue with a predicted IC50 value of 500nm or less (as described in Espinasa et al, 2013) were used to generate the positive test set. Matched negatives were then identified as described above. The test set contained 158 peptides in total; 78 that were isolated from 10 different class I HLA/MHC alleles and their 78 matched negative partners.

### Sample10 test set

10 positive and 10 negative peptides for each allele were randomly selected and removed from the training data and used for subsequent testing. Note: for alleles where less than 10 positive and negative peptides were available the maximum number available were selected and removed. The final test set contained 608 peptides in total; 304 that were isolated from 31 different class I alleles and their 304 matched negative partners.

### Sample10 complementary test set

The nonomeric class I peptides that were excluded from the training data as they had a predicted IC₅₀ value of greater than 500nm were used to form a positive "weak-binding" test set. Matched negatives were then identified as described above. The final test set contained 5200 peptides in total; 2600 that were isolated from 30 different class I HLA/MHC alleles and their 2600 matched negative partners.

### Training data validation testing

### 3-fold cross validation

3-fold cross validation was routinely performed to evaluate different training set compositions and different training features. In such experiments the training data was randomly partitioned into 3 different complementary subsets. 2 of the 3 subsets were used for training while the remaining subset was used for subsequent testing. The cross validation process was then repeated, with each subset being used once for testing. The overall all results for each of the 3 rounds of testing were then averaged to produce a single performance metric

### Conventional validation

In addition, conventional validation was performed, where the training data was partitioned into 2 sets; one contained 70% of the peptides and was used for training and the other contained 30% of the peptides and was used for testing.

### Evaluation of SVM model performance.

To assess the prediction accuracy of the SVM model, we used the area under the ROC (receiver operating characteristic) curve otherwise known as AUC, which provides a classifiers recall and specificity by plotting the recall (true positives) and 1- specificity (true negatives) as a function of this threshold (Bradley et al, 1997). The AUC is a threshold independent metric obtained by the area under the ROC curve. The AUC score ranges between 0 and 1, the former indicates a total inverse prediction, the latter stands for perfect prediction, and 0.5 means a random prediction.

### Results

### Example 1 - Advantage of using matched pairs from same source protein, and subsequent optimization of the matched pair training set.

In order to investigate the benefit of selecting the matching negative from the same protein as the positive, different training sets were generated where the matching negative member of each pair was selected from the same or a random protein. The negative peptide was selected on the basis of it sharing a predicted binding affinity within a 10%, 100% or 10-100% range of its respective positive partner. The different training sets were then used to train a SVM algorithm, using VHSE and vector frequency (dimers) as training features across the whole peptide length and 3 amino-acid long peptide flanking regions extracted from the parental protein (subsequently referred to as the "Wide" configuration).

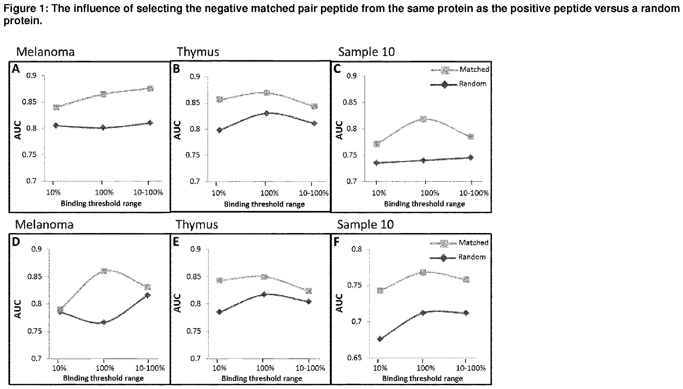

Each algorithm was then tested using three different independent test sets referred to as the Melanoma, Thymus & Sample10 test sets. The results for the different test sets (measured using AUC) are shown in Figure1 (panels A, B & C respectively). The Figure clearly shows that selecting the negative peptide from the same protein as the positive (rather than a random protein) generates a significant improvement in performance ranging from 1-9%. Interestingly, the optimal binding range for selecting negative peptides appears to be in the range of 0-100%.

The experiments were repeated but the anchor regions (positions 2 & 9 in the nonomer) were excluded as training features for algorithm training (Excluded), and the results for the three datasets (Melanoma, Thymus and Sample10) are shown in panels D, E & F respectively. While the AUC measurements for the later experiment were slightly lower than those reported previously using the Wide feature set, the fact that the removal of the anchors did not destroy the performance completely suggests that the algorithm has "learnt" features associated with efficient presentation rather than HLA/MHC binding and is thus operating in an HLA/MHC agnostic manor.

### Example 2 - Investigating the influence of the predicted binding affinity differential between the positive and negative members of the training set on performance.

In order to investigate the relationship between the positive and negative members of a matched pair used for training, different training sets were generated where the matching negative members were selected on the basis outlined in the table below; creating training sets with increasingly wide binding differentials between the positive and negative members.

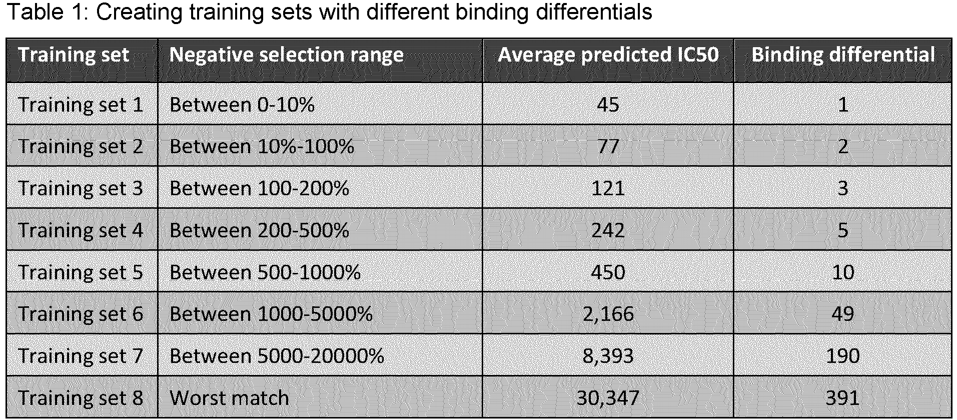

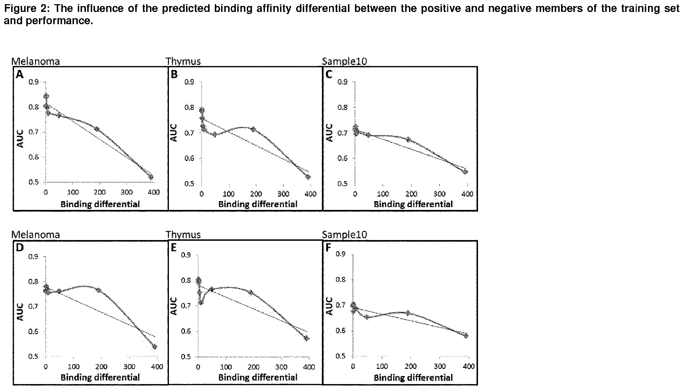

Once the training sets were generated they were equalised in terms of size by only selecting matching pairs where the positives were common to all the different groups. The equalised training sets were subsequently used to train 8 different SVM algorithms (using the training features described above). Each algorithm was then tested using the Melanoma, Thymus & Sample10 test sets and the results shown in Figure 2 (panels A, B & C respectively). The results demonstrate that as the binding differential increases above 3 the performance of the algorithm begins to fall, as it presumably begins to "learn" features associated with binding as well as processing. Trend lines are shown in black. Interestingly while the performance on the independent balanced test sets deteriorated as the binding differential increased the cross validation score increased from 0.72 to 0.985. This reciprocal relationship strongly suggesting that as the binding differential increases the algorithm begins to learn features associated with HLA/MHC binding rather than processing and presentation, and by the time the differential has reached 400 the classifier is only recognising features associated with binding (as the independent test set performance has fallen to AUC 0.52 versus 0.985 for the cross validation).

The experiments were repeated using the Excluded feature set described above. Each algorithm was then tested using the Melanoma, Thymus & Sample10 test sets and the results shown in Figure 2 (panels D, E & F respectively). Interestingly, while the curves for the "excluded"-trained algorithms follow the same overall trend as those trained using the Wide feature set, the decline in performance is delayed, as exclusion of the anchor regions appears to help offset the effect of the increasing binding differential i.e. delays the point at which the algorithm begins to learn features associated with binding as well as processing. This hypothesis is supported by the observation that the cross validation score increased more slowly when the Excluded feature set was used for training compared to the Wide feature set and peaked at 0.923 versus 0.985. This observation provides further evidence that machine-learning algorithms trained with the method described herein (using both the Wide and Excluded feature sets) "learn" the features associated with efficient presentation rather than HLA/MHC binding and can operate in an HLA/MHC agnostic manor.

### Example 3 - Optimizing the composition of the negative training set to improve performance.

In order to find the optimal criteria for selecting the negative training set, we created a series of negative datasets where the negative peptide was selected on the basis of it sharing a predicted binding affinity within a pre-defined range of its respective matching positive partner as defined in table 2 below.

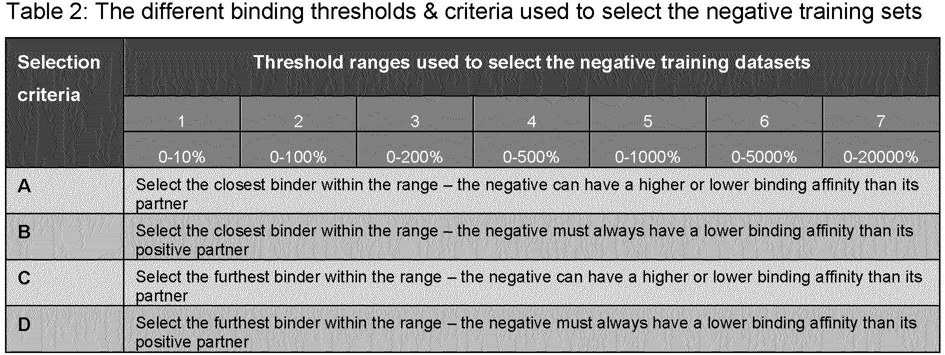

The 28 different training sets were then used to train SVM algorithms. Each algorithm was then tested using the Sample10 test set (where all the positive peptides had a predicted binding IC₅₀ value below 500nm) and the sample 10 complementary test set (where all the positive peptides had a predicted binding IC₅₀ value above 500nm) which contained 608 and 5200 peptides respectively.

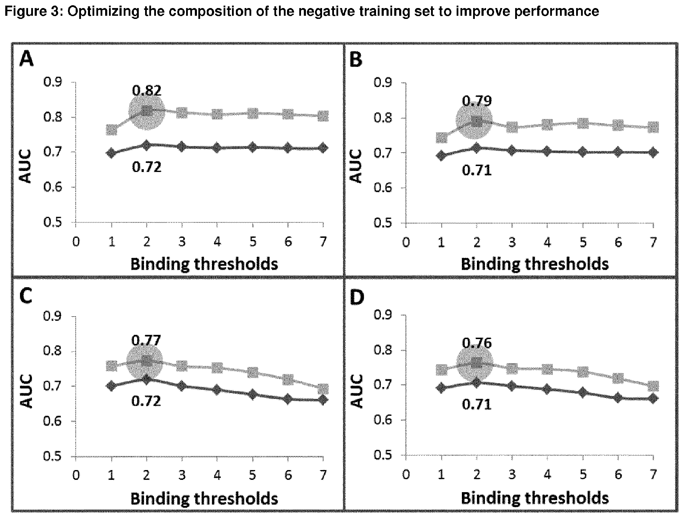

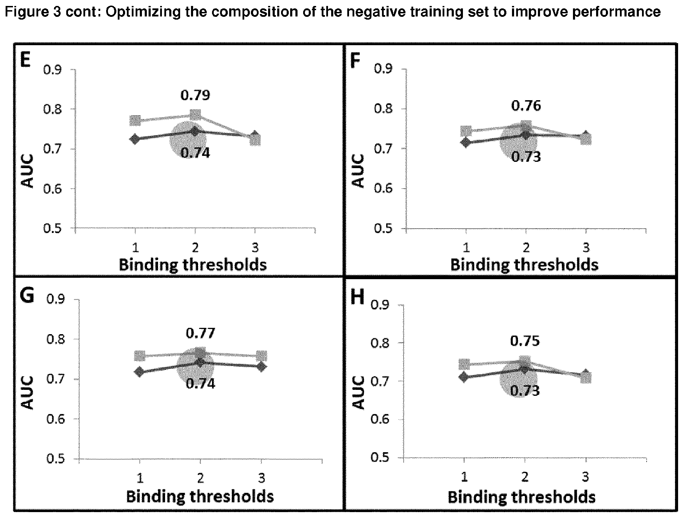

As shown in Figure 3 panels A-D (red line) the optimal binding threshold for selecting negative peptides appears to be in the range of 0-100% (where the negative peptide is selected on the basis of it having either a higher or lower binding affinity than its positive partner) for the Sample10 test set with an AUC measurement of 0.82 which represented an improvement in performance ranging from 3-6% compared with the other trained algorithms (see red line in panels B-D). A similar trend was observed with the sample 10 complementary test set although the differences in performance were more modest (see blue line panels A-D).

The above experiments were repeated except the series of negative datasets were created using mutually exclusive ranges of affinity matched negatives (bins), rather than "sliding scale" thresholds as shown in table 3 below:

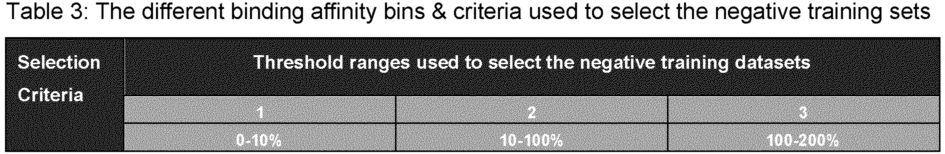

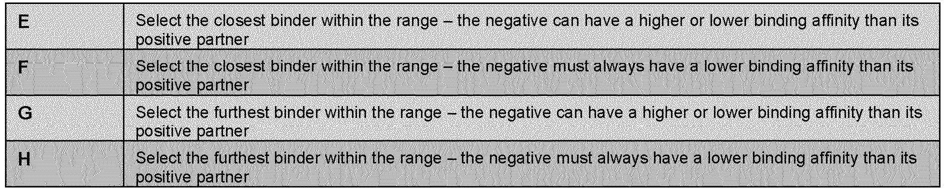

As shown in Figure 3 panel E (blue line) compared to panels F-H the optimal binding threshold for selecting negative peptides was in the range of 10-100% (where the negative peptide can have a higher or lower binding affinity than its positive partner) for both test sets. However, while the optimal performance for the Sample10 test set was lower than that reported using a binding scale thresholds of 1-100 (0.82 versus 0.79), the performance for the sample 10 complementary test set was actually higher (0.74 versus 0.72). This suggests that the use of a mutually exclusive binding range may be better for training machine-learning algorithms than the use of a sliding scale range, to classify processed peptides that have a weaker binding affinity for their respective HLA/MHC molecule (peptides with an IC₅₀ below above 500nm).

### Example 4 - Demonstrating the allele agnostic nature the matched pair approach

In order to demonstrate that the matched-pair method described herein can be used to train a machine-learning algorithm to identify peptides that contain features associated with processing and presentation and not HLA/MHC binding, and thus can be applied to any peptide regardless of its MHC restriction, i.e. the algorithm is HLA/MHC-agnostic, we trained and tested an SVM algorithm for each individual allele represented in our training set as outlined in the table below:

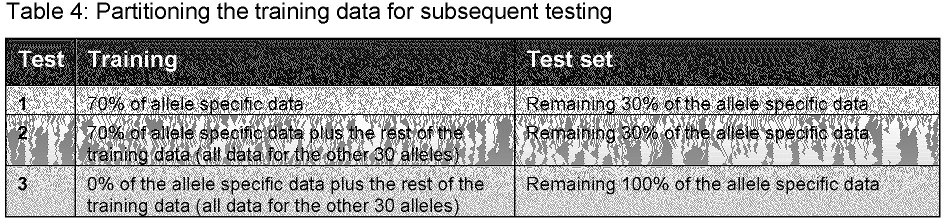

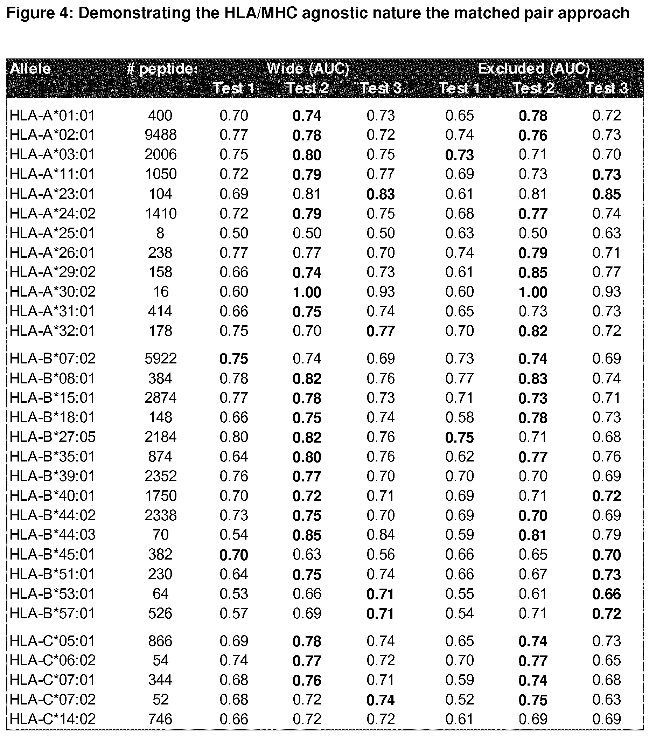

As shown in figure 4 the results clearly demonstrate that the matched-pair trained SVM classifier regularly makes equivalent or better predictions when trained in a non HLA/MHC-allele specific manner (tests 2 and 3) compared to when it is trained in an allele-specific manner (tests 1). This trend is observed for algorithms trained using both the Wide and Excluded feature sets.

### Example 5 - Benchmarking against NetChop3 (the only other HLA/MHC-agnostic processing tool commonly used)

A SVM algorithm was trained using the optimized training set: where negative peptides were identified from the same parental protein as their positive counterpart and selected on the basis of having an estimated IC₅₀ binding affinity within a 100% range of the matching positive. The algorithm was also trained using VHSE and frequency vector (dimers) as training features across the whole peptide length and 3 amino-acid long flanking regions (wide), the resulting algorithm was named PanPro (Wide). A second algorithm was trained on the exact same training set using the same training features, except that the anchor regions were excluded as training features (Excluded), the resulting algorithm was named PanPro (Excluded).

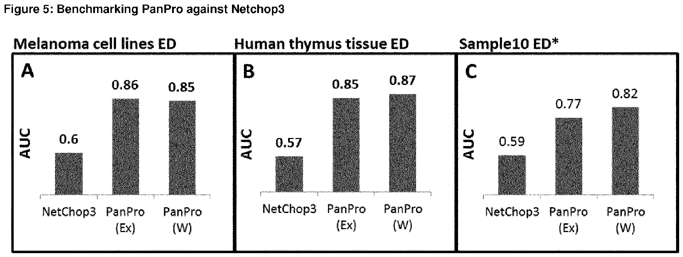

Each algorithm was then benchmarked against NetChop-termC 3.0 using the Melanoma, Thymus & Sample10 test sets . As shown in Figure 5 (panels A-C) both versions of PanPro outperformed NetChop-termC3.0 across all three test sets. The biggest difference in performance was in PanPro's ability to correctly call negatives leading to a low false positive rate (data not shown).

### Example 6 - Benchmarking PanPro against HLA/MHC-specific classifiers MHC-NP (demonstrating that our pan approach can compete with the current gold standard HLA/MHC-specific trained methods).

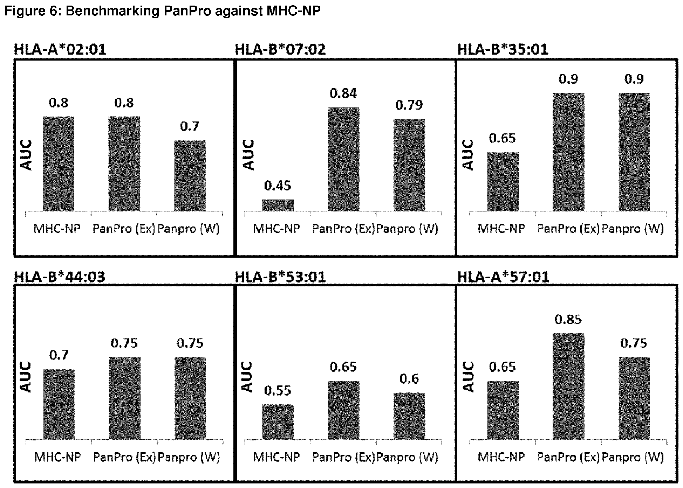

PanPro trained using the "Excluded" and "Wide" feature sets described previously were compared with MHC-NP (Giguere et al. 2013) using the relevant allele specific test data extracted from the Sample10 test set. As shown in Figure 6 both versions of PanPro outperformed MHC-NP for 5 out of the 6 alleles tested.

### Discussion

Less than 15% of validated HLA/MHC binding peptides are naturally processed and have an opportunity to interact with a T-cell (Giguere et al. 2013), and less than 5% are capable of eliciting an immune response. (Robbins et al, 2013). Thus there is a clear need to develop *in silico* methods for identifying peptides that will be naturally processed, which can be combined with HLA/MHC binding predictors to improve the ability to identify immunogenic antigens in a timely and cost effective manner. Unfortunately the performance of algorithms trained to learn the features of processing and presentation lag those of HLA/MHC binding predictors (Giguere et al. 2013). One of the challenges to developing *in silico* methods is the complexity of the processing and presentation pathways, which involves multiple steps and multiple proteases, chaperones and transport proteins *etc.* (Neefjes et al. 2011). Another challenge is that multiple "sequence-independent" factors influence whether a peptide is likely to be naturally processed including the abundance and stability of the source protein. Thus peptides that contain the right physiochemical properties to be efficiently processed and presented may never be observed bound to HLA/MHC at the cell surface as the source protein lacks the necessary characteristics. Finally, untangling the features of naturally processed peptides that are necessary for efficient processing and presentation rather than HLA/MHC binding has proven challenging; as the features that contribute to binding, especially the anchor regions, tend to dominate the information landscape, a problem that is exacerbated by the fact that these processes have co-evolved and the relevant physiochemical features probably overlap (Kesmir et al. 2003). In this patent we describe a method for training a machine-learning algorithm or statistical inference model that controls for the influence of protein abundance, stability and HLA/MHC binding, enabling the algorithm or model to learn features that are synonymous with efficient processing and presentation, rather than HLA/MHC binding. As the influence of the HLA/MHC binding is negated the algorithm or model can be applied to any peptide regardless of its HLA/MHC restriction.

The results clearly show that there is an advantage in building a paired negative dataset where the negative members are selected on the basis that they originate from the same source protein as their positive counterpart (controls for differences in protein abundance and stability) see Figure 1, and share a similar HLA/MHC binding affinity with respect to the same HLA/MHC allele (controls for the influence of HLA/MHC binding) see Figures 2 & 3. In addition, we have experimented with excluding the anchor positions 2 and 9 as features for machine learning, in order to further minimise any influence of HLA/MHC binding. Interestingly, while the algorithms trained on this partial peptide sequence (Excluded) performed slightly less well than those trained on the full peptide (Wide) the drop in performance is relatively small - further supporting our hypothesis that the algorithm has learnt the features associated with processing rather than HLA/MHC binding, as removal of the anchor regions would destroy the performance of a HLA/MHC binding predictor.

Furthermore, as structuring the training data in this manner enables the machine-learning algorithm to learn the true universal features that are associated with efficient processing and presentation, it can be applied to any peptide regardless of its HLA/MHC restriction i.e. the algorithm or model operates in an HLA/MHC-agnostic manner see figure 4.

Finally, we have trained two SVM algorithm using the method described herein utilising the Wide and Excluded feature sets and using the VHSE and frequency vector (dimers) as training features and called the algorithms PanPro (Wide) and PanPro (Excuded), and benchmarked the performance against NetChop-termC-3. Interestingly, both versions of PanPro significantly outperformed NetChop-termC-3. We also benchmarked the performance of PanPro against the allele-specific processing prediction tool MHC-NP. Both versions of PanPro out-performed MHC-NP in relation to 5 out of the 6 alleles tested, with PanPro (Excuded) performing the strongest.

To conclude, we believe that we have developed the first machine-learning based classifier that has learnt the true physiochemical features that determine efficient processing and presentation. We have shown that the algorithm can be used to evaluate any peptide regardless of its MHC restriction, and is thus HLA/MHC-agnostic. The classifier should operate synergistically with HLA/MHC binding algorithms to help improve the ability to identify immunogenic antigens *in silico.*

### References

Bradly et al. (1997). The use of the area under the ROC curve in the evaluation of machine learning algorithms. Pattern Recognition, 30(7):1149-1155
Emmerich et al. (2000). The human 26 S and 20 S proteasomes generate overlapping but different sets of peptide fragments from a model protein substrate. J Biol Chem. 2000 Jul 14;275(28):21140-8.
Espinosa et al. (2013). Peptides presented by HLA class I molecules in the human thymus. J Proteomics. 94: 23-36
Giguere et al. (2013). MHC-NP: predicting peptides naturally processed by the MHC. J Immunol Methods. 2013 Dec 31;400-401:30-6
Ginodi et al. (2008). Precise score for the prediction of peptides cleaved by the proteasome. Bioinformatics. 2008 Feb 15;24(4):477-83.
Holzhutter & Kloetzel (2000). A kinetic model of vertebrate 20S proteasome accounting for the generation of major proteolytic fragments from oligomeric peptide substrates. Biophys J. 2000 Sep;79(3):1196-205
Jarmalavicius et al. (2012). High Immunogenicity fo the Human Leukocyte Antigen Pepidomes of Melanoma Tumor Cells. J Biol Chem. 287, 40: 33401-33411.
Mei et al. (2005). A new set of amino acid descriptors and its application in peptide QSARs. Biopolymers. 80, 6:775-86.
Kesmir et al. (2002). Prediction of proteasome cleavage motifs by neural networks. Protein Eng. 2002 Apr;15(4):287-96.
Kesmir et al. (2003). Bioinformatic analysis of functional differences between the immunoproteasome and the constitutive proteasome. Immunogenetics 55: 437-449.
ZhiLiang et al. (2008). A novel descriptor of amino acids and its application in peptide QSAR. Journal of Theoretical Biology 253(1):90-7 August 2008
Mei et al. (2005). A new set of amino acid descriptors and its application in peptide QSARs. Biopolymers. 2005;80(6):775-86.
Neefjes et al. (2011). Towards a systems understanding of MHC class I and MHC class II antigen presentation. Nat Rev Immunol. 2011 Nov 11;11(12):823-36.
Nielsen et al. (2005). The role of the proteasome in generating cytotoxic T-cell epitopes: insights obtained from improved predictions of proteasomal cleavage. Immunogenetics. 2005 Apr;57(1-2):33-41.
Nussbaum et al. (2001). PAProC: a prediction algorithm for proteasomal cleavages available on the WWW. Immunogenetics. 2001 Mar;53(2):87-94.
Robins et al. (2013). Mining exomic sequencing data to identify mutated antigens recognized by adoptively transferred tumor-reactive T cells. Nat Med. 2013 Jun;19(6):747-52
Tang et al. (2014). NIEluter: Predicting peptides eluted from HLA class I molecules. J Immunol Methods. 2015 Jul;422:22-7.
Tenzer et al. (2004). Quantitative analysis of prion-protein degradation by constitutive and immuno-20S proteasomes indicates differences correlated with disease susceptibility. J Immunol. 2004 Jan 15;172(2):1083-91
Tenzer & Schild (2005). Assays of proteasome-dependent cleavage products. Methods Mol Biol. 2005;301:97-115.
The UniProt Consortium (2014). Activities at the Universal Protein Resource (UniProt) Nucleic Acids Res. 42: D191-D198 (2014).
Toes et al. (2001). Discrete cleavage motifs of constitutive and immunoproteasomes revealed by quantitative analysis of cleavage products. J Exp Med. 2001 Jul 2;194(1):1-12.

### Clauses

The disclosure may be further understood with reference to the following numbered clauses.

Clause 1. A method for training a machine learning algorithm or statistical inference model to identify peptides that contain features positively associated with natural endogenous or exogenous cellular processing, transportation and major histocompatibility complex (MHC) presentation, that negates the influence of HLA/MHC-binding and can be applied to any peptide regardless of its MHC restriction, comprising:
(a) building one or more training data sets comprising a positive and a negative data set;
   wherein the positive data set comprises entries of peptide sequences identified or inferred from surface bound or secreted HLA/MHC/peptide complexes encoded by one or a plurality of different HLA/MHC alleles, and wherein the negative data set comprises entries of peptide sequences which are not identified or inferred from surface bound or secreted HLA/MHC/peptide complexes;
   wherein the training data further comprises a multiplicity of pairings between entries of the positive and negative data sets; and wherein each pair of said multiplicity of pairings comprises peptide sequences which:
      (i) are of equal or similar length,
         and
      (ii) are derived from the same source protein or fragment thereof,
         and/or
      (iii) have similar binding affinities, with respect to the HLA/MHC molecule which the positive counterpart is restricted,
and (b) applying a machine learning algorithm or statistical inference model on said training data.

Clause 2. A method according to clause 1, wherein each pair of said multiplicity of pairings comprises of peptide sequences which fulfil criteria (i), (ii) and (iii).

Clause 3. A method according to clause 1, or 2, wherein the amino acids at key HLA/MHC-binding anchor positions within the peptide sequences of the positive and negative data sets are removed as features for a machine learning algorithm or statistical inference model.

Clause 4. A method according to clause 1, 2 or 3 wherein step (b) comprises applying a machine learning algorithm on said training data.

Clause 5. A method according to clause 4, wherein the machine learning algorithm is supervised.

Clause 6. A method according to clause 4, wherein the machine learning algorithm is unsupervised

Clause 7. A method according to any preceding clause, wherein the positive data set comprises entries of peptide sequences identified or inferred from surface bound or secreted HLA/MHC/peptide complexes encoded by a plurality of different HLA/MHC alleles.

Clause 8. A method according to any preceding clause, wherein the positive data set comprises peptide sequences identified or inferred from at least 2, preferably at least 20, more preferably at least 50, different surface-bound or secreted HLA/MHC variants encoded by different HLA/MHC alleles.

Clause 9. A method according to any preceding clause, wherein the positive data set comprises peptide sequences identified or inferred from surface bound or secreted HLA/MHC variants encoded by (a) HLA/MHC Class I alleles of either the HLA -A, -B, or -C gene loci, or equivalent loci thereof in a non-human species, or any combination thereof, or (b) MHC Class II alleles of either the HLA -DQ, -DP, or -DR gene loci, or equivalent loci thereof in a non-human species, or any combination thereof; wherein the positive data set is derived from the same species.

Clause 10. A method according to any preceding clause, wherein said positive data set comprises peptide sequences identified or inferred from all of said gene loci according to (a), or all of said gene loci according to (b).

Clause 11. A method according to any preceding clause, wherein each peptide sequence of both the positive and negative data sets is of equal length; preferably wherein said length is 8, 9, 10, 11, or greater than 11 amino acids.

Clause 12. A method according to any preceding clause, wherein said binding affinity of each matching negative peptide, when measured using the IC₅₀ nm metric, differs by no more than (in increasing preference) 500%, 200%, and 100%, compared to the binding affinity of its positive counterpart.

Clause 13. A method according to any preceding clause, wherein said estimated binding affinities have been obtained via an MHC binding prediction algorithm, experimental measurement or combinations thereof.

Clause 14. A method according to any preceding clause, wherein amino acid identity, size, charge, polarity, hydrophobicity and/or other relevant physicochemical property at a given position in peptide sequences of the positive and negative data sets are used as features for said machine learning algorithm or statistical inference model.

Clause 15. A method according to any preceding clause, wherein the peptide sequences are represented as concatenated vectors and wherein each amino acid is encoded as a binary vector with one element for each possible amino acid, wherein the presence of each amino acid is denoted with a 1 and the absence of each amino acid is denoted with a 0.

Clause 16. A method according to any preceding clause, wherein amino acid identity, charge, size, polarity, hydrophobicity and/or other relevant physicochemical property in positions which, in the source protein, are within 10, preferably 5 or more preferably 3 positions of the termini of the peptide sequences of the positive and negative data sets are used as features for said machine learning algorithm or statistical inference model.

Clause 17. A method according to any preceding clause, wherein the positive and negative data sets further comprise principle component score vectors of hydrophobic, steric and electronic property (VHSE) descriptors for the amino acids of peptide sequences in said data sets; and wherein said descriptors are used as features for said machine learning algorithm or statistical inference model.

Clause 18. A method according to any preceding clause, wherein the positive and negative data sets further comprise principle component score vectors of topological and structural property (VTSA) descriptors for the amino acids of peptide sequences in said data sets; and wherein said descriptors are used as features for said machine learning algorithm or statistical inference model.

Clause 19. A method according to any preceding clause, wherein the *k-mer* frequency of an amino acid sequence at a given position in the peptide sequences of the positive and negative data sets are used as features for said machine learning algorithm or statistical inference model; wherein k is equal to 1, 2 or 3.

Clause 20. A method according to any preceding clause, further comprising, following step (b), interrogating input data comprising amino acid sequences of peptides and/or proteins with said machine learning model, to identify peptides, or peptide fragments of said proteins, having features positively associated with natural endogenous or exogenous cellular processing, transportation and HLA/MHC presentation.

Clause 21. A computer readable medium having computer executable instructions stored thereon for implementing the method of any of clauses 1 to 20.

Clause 22. An apparatus comprising:
one or more processors; and
memory comprising instructions which when executed by one or more of the processors cause the apparatus to perform the method of any of clauses 1-20.

## Claims

1. A computer-implemented method, comprising:
building one or more training data sets comprising a positive and a negative data set;
wherein the positive data comprises entries of peptide sequences identified or inferred from surface bound or secreted HLA/MHC/peptide complexes encoded by one or a plurality of different HLA/MHC alleles, and wherein the negative data comprises entries of peptide sequences which are not identified or inferred from surface bound or secreted HLA/MHC/peptide complexes;
wherein the training data further comprises a multiplicity of pairings between entries of the positive and negative data sets; and wherein each pair of said multiplicity of pairings comprises peptide sequences which:
(i) are of equal or similar length,
and
(ii) are derived from the same source protein or fragment thereof,
and/or
(iii) have binding affinities which differ by no more than 500%, with respect to the HLA/MHC molecule which the positive counterpart is restricted,
wherein each peptide sequence is encoded as a feature vector and the feature vector is an n-dimensional vector of numerical parameters that represent that peptide sequence; and,
training a machine learning algorithm or statistical inference model on the multiplicity of pairings to receive input data comprising sequences of peptides, whole proteins or fragments thereof, such sequences broken into peptides of equal or similar length to the peptide sequences of the multiplicity of pairings, and provide outputs classified into one of two categories: processed and presented on the cell surface or not processed or presented on the cell surface, or converted into a probabilistic scale using mathematical techniques, creating a trained algorithm to identify peptides that contain features positively associated with natural endogenous or exogenous cellular processing, transportation and major histocompatibility complex (MHC) presentation, that negates the influence of HLA/MHC-binding and can be applied to any peptide regardless of its MHC restriction.

2. A computer-implemented method, comprising:
interrogating input data comprising amino acid sequences of peptides and/or proteins with a machine learning model or statistical inference model, to identify peptides, or peptide fragments of said proteins, having features positively associated with natural endogenous or exogenous cellular processing, transportation and HLA/MHC presentation,
wherein outputs of the machine learning model or statistical inference model are configured to be classified into one of two categories: processed and presented on the cell surface or not processed or presented on the cell surface, or converted into a probabilistic scale using mathematical techniques,
wherein the machine learning model or statistical inference model has been trained on one or more training data sets comprising a positive and a negative data set;
wherein the positive data set comprises entries of peptide sequences identified or inferred from surface bound or secreted HLA/MHC/peptide complexes encoded by one or a plurality of different HLA/MHC alleles, and wherein the negative data set comprises entries of peptide sequences which are not identified or inferred from surface bound or secreted HLA/MHC/peptide complexes;
wherein the training data further comprises a multiplicity of pairings between entries of the positive and negative data set; and wherein each pair of said multiplicity of pairings comprises peptide sequences which:
(i) are of equal or similar length,
and
(ii) are derived from the same source protein or fragment thereof,
and/or
(iii) have binding affinities which differ by no more than 500%, with respect to the HLA/MHC molecule which the positive counterpart is restricted,
wherein each peptide sequence is encoded as a feature vector and the feature vector is an n-dimensional vector of numerical parameters that represent that peptide sequence; and,
wherein the input data comprises sequences of peptides, whole proteins or fragments thereof, such sequences broken into peptides of equal or similar length to the peptide sequences of the multiplicity of pairings.

3. A method according to claim 1 or 2, wherein each pair of said multiplicity of pairings comprises of peptide sequences which fulfil criteria (i), (ii) and (iii).

4. A method according to any of claims 1 to 3, wherein the amino acids at key HLA/MHC-binding anchor positions within the peptide sequences of the positive and negative data sets are removed as features for a machine learning algorithm or statistical inference model.

5. A method according to any preceding claim, wherein the positive data set comprises peptide sequences identified or inferred from at least 2, preferably at least 20, more preferably at least 50, different surface-bound or secreted HLA/MHC variants encoded by different HLA/MHC alleles.

6. A method according to any preceding claim, wherein the positive data set comprises peptide sequences identified or inferred from surface bound or secreted HLA/MHC variants encoded by (a) HLA/MHC Class I alleles of either the HLA -A, -B, or -C gene loci, or equivalent loci thereof in a non-human species, or any combination thereof, or (b) MHC Class II alleles of either the HLA -DQ, -DP, or -DR gene loci, or equivalent loci thereof in a non-human species, or any combination thereof; wherein the positive data set is derived from the same species.

7. A method according to any preceding claim, wherein each peptide sequence of both the positive and negative data sets is of equal length; preferably wherein said length is 8, 9, 10, 11, or greater than 11 amino acids.

8. A method according to any preceding claim, wherein said binding affinity of each matching negative peptide, when measured using the IC₅₀ nm metric, differs by no more than (in increasing preference), 200%, 100%, and 10% compared to the binding affinity of its positive counterpart.

9. A method according to any preceding claim, wherein amino acid identity, size, charge, polarity, hydrophobicity and/or other relevant physicochemical property at a given position in peptide sequences of the positive and negative data sets are used as features for said machine learning algorithm or statistical inference model.

10. A method according to any preceding claim, wherein the peptide sequences are represented as concatenated vectors and wherein each amino acid is encoded as a binary vector with one element for each possible amino acid, wherein the presence of each amino acid is denoted with a 1 and the absence of each amino acid is denoted with a 0.

11. A method according to any preceding claim, wherein amino acid identity, charge, size, polarity, hydrophobicity and/or other relevant physicochemical property in positions which, in the source protein, are within 10, preferably 5 or more preferably 3 positions of the termini of the peptide sequences of the positive and negative data sets are used as features for said machine learning algorithm or statistical inference model.

12. A method according to any preceding claim,
wherein the positive and negative data sets further comprise principle component score vectors of hydrophobic, steric and electronic property (VHSE) descriptors for the amino acids of peptide sequences in said data sets; and wherein said descriptors are used as features for said machine learning algorithm or statistical inference model; and/or wherein the positive and negative data set further comprise principle component score vectors of topological and structural property (VTSA) descriptors for the amino acids of peptide sequences in said data sets; and wherein said descriptors are used as features for said machine learning algorithm or statistical inference model; and/or
wherein the *k-mer* frequency of an amino acid sequence at a given position in the peptide sequences of the positive and negative data sets are used as features for said machine learning algorithm or statistical inference model; wherein k is equal to 1, 2 or 3.

13. A method according to any preceding claim, wherein the positive data comprises entries of naturally processed peptide sequences identified in an elution assay and wherein the negative data comprises entries of not naturally processed peptide sequences not observed in an elution assay.

14. A computer readable medium having computer executable instructions stored thereon for implementing the method of any of claims 1 to 13.

15. An apparatus comprising:
one or more processors; and
memory comprising instructions which when executed by one or more of the processors cause the apparatus to perform the method of any of claims 1 to 13.
